# FASCICULE DE BREVET EUROPEEN

(11) **EP 2 307 060 B1**
(45) Date de publication et mention de la délivrance du brevet: **25.04.2018**
(21) Numéro de dépôt: 09797557.7
(22) Date de dépôt: 22.06.2009
(51) Int. Cl.: A61L 27/12, A61L 27/36, A61L 27/24, A61L 24/00

(54) **COMBINAISON DE SANG ET DE PARTICULES DE CERAMIQUE DE PHOSPHATES DE CALCIUM BIPHASES**
KOMBINATION AUS BLUT UND BIPHASISCHEN CALCIUMPHOSPHAT-KERAMIKPARTIKELN
COMBINATION OF BLOOD AND OF BIPHASIC CALCIUM PHOSPHATE CERAMIC PARTICLES

(30) Priorité: 23.06.2008 FR 0803492
(43) Date de publication de la demande: 13.04.2011
(73) Titulaire: Centre National de la Recherche Scientifique, 75016 Paris (FR); Centre Hospitalier Universitaire de Nice, 06003 Nice Cedex 01 (FR)
(72) Inventeur: BALAGUER, Thierry, 06670 Colomars (FR); ROCHET, Nathalie, 06100 Nice (FR); TROJANI, Christophe, 06950 Falicon (FR); BOUKHECHBA, Florian, 06000 Nice (FR); CARLE, Georges, 06000 Nice (FR)
(74) Mandataire: Corizzi, Valérie
(86) Numéro de dépôt international: PCT/FR2009/000749
(87) Numéro de publication internationale: WO 2010/007230

(56) Documents cités:
- WO-A-01/81243
- WO-A-02/068010
- WO-A-2006/015275
- WO-A-2006/058153
- HING K A ET AL: "Microporosity enhances bioactivity of synthetic bone graft substitutes" JOURNAL OF MATERIALS SCIENCE: MATERIALS IN MEDICINE, KLUWER ACADEMIC PUBLISHERS, BO, vol. 16, no. 5, 1 mai 2005 (2005-05-01), pages 467-475, XP019212185 ISSN: 1573-4838
- Olivier Malard ET AL: "Influence of biphasic calcium phosphate granulometry on bone ingrowth, ceramic resorption, and inflammatory reactions: Preliminaryin vitro andin vivo study", JOURNAL OF BIOMEDICAL MATERIALS RESEARCH, vol. 46, no. 1, 1 July 1999 (1999-07-01), pages 103-111, XP055337286, US ISSN: 0021-9304, DOI: 10.1002/(SICI)1097-4636(199907)46:1<103::A ID-JBM12>3.0.CO;2-Z

## Description

L'invention a pour objet un nouveau biomatériau à base de sang coagulé ou d'aspirat de moelle osseuse coagulé, et de particules de céramique de phosphates de calcium biphasés, un procédé pour sa préparation et son utilisation pour la fabrication d'un implant pour permettre la régénération du tissu osseux.

La reconstruction des pertes de substance osseuse, d'origine principalement traumatique et plus rarement tumorale, est une des grandes difficultés rencontrées par les chirurgiens orthopédistes. Les défauts de petite taille, depuis la pseudarthrose « serrée » (défaut de consolidation d'une fracture où la perte de substance est virtuelle) jusqu'à des pertes osseuses de 5-6 cm font, le plus souvent, l'objet d'une greffe autologue de tissu osseux spongieux ou cortico-spongieux prélevé sur la crête iliaque (gold standard). Les défauts de grandes tailles (≥ 6 cm) nécessitent des interventions beaucoup plus lourdes, transferts osseux vascularisés ou procédure de Masquelet. Malgré tout, la quantité d'os autologue disponible est limitée, la consolidation osseuse reste aléatoire et ces différentes techniques sont fortement pourvoyeuses de complications post opératoires au niveau du site de prélèvement de la greffe.

Différents biomatériaux disponibles en pratique clinique permettent d'éviter, en théorie, les inconvénients de la greffe autologue. Malheureusement, aucun d'eux n'égale les résultats de la greffe osseuse et ils ne permettent jamais la reconstruction de pertes de substance de grande taille.

La majorité des substituts osseux étudiés actuellement associent aux biomatériaux des cellules souches mésenchymateuses obtenues à partir de moelle osseuse après plusieurs semaines de sélection et de culture cellulaire *in vitro.* Cette approche est lourde et coûteuse, ce qui limite les retombées cliniques.

L. Okazaki et al., Clin. Oral Impl. Res., 16, 2005, 236-243 décrit des implants à base soit de poudre d'os déminéralisé, soit de sang coagulé. Plusieurs auteurs ont étudié l'association de sang avec des biomatériaux de synthèse : J. Schmid et al., Clin. Oral Impl. Res. 1997:8:75-8 décrit des implants à base de poudre d'os de bovin déminéralisé et de sang. A. Chevrier et al., Osteoarthritis and Cartilage (2007), 15, 316-327, décrit des implants à base d'une solution de polymère contenant du chitosan dans un tampon glycérol-phosphate et de sang qui coagule *in situ.* B. Wallkamm et al., Clin. Oral Imp. Res., 14, 2003, 734-742 décrit des implants à base de sang et d'un dérivé d'acide polylactique (Polyfïbre® ou Polyfoam®). Yildérim M. et al., Clin. Oral Impl. Res., 2000, 11, 217-219 décrit des implants à base d'apatite bovine et de sang veineux. Le document US 2008/0014279 décrit un biomatériau constitué d'un matériau granulaire recouvert d'un gel de polymère, et qui peut être mélangé à toute sorte de liquide, notamment du sang, pour former une pâte qui est appliquée à la spatule ou à la seringue là où un défaut osseux doit être comblé. Le document WO 02/068010 décrit un matériau composite à base de moelle osseuse, ce matériau comprenant une matrice implantable biocompatible et poreuse et un matériau coagulé, tel qu'un coagulat de moelle osseuse, de sang, de plasma. WO2006/058153 décrit des implant osseux comprenant un agent coagulant, e.g. le chlorure de calcium mélangé avec un matériau de réparation osseuse (par exemple un phosphate de calcium) et du sang ou un aspirat de moelle osseuse. Toutefois, aucun de ces matériaux implantables décrit dans l'art antérieur n'est dépourvu d'inconvénients :
Les matériaux nécessitant la mise en culture de cellules de moelle osseuse avant une association avec un support (os déminéralisé, polymère de synthèse ou autre) sont longs à mettre en oeuvre et contraignent à faire un prélèvement de moelle osseuse sur l'individu à traiter plusieurs semaines avant la pose de l'implant, ce qui multiplie les interventions et les risques qui y sont associés.

Les biomatériaux associant un support et du sang non coagulé ne permettent pas de construire un implant.

Si l'association de certains matériaux supports avec du sang coagulé a été proposée, les résultats obtenus ne sont pas toujours satisfaisants, notamment parce que le procédé ne permet pas l'obtention d'un biomatériau homogène.

De tels matériaux résultant de l'association d'un support et de sang, coagulé ou non, ont été jusqu'à présent utilisés en chirurgie maxillo-faciale où les problèmes de consolidation osseuse sont moins critiques, mais ils ont été peu ou pas utilisés dans la réparation d'os diaphysaires.

Dans le cas de WO 02/068010, le procédé enseigné consiste à utiliser un matériau support constitué d'os déminéralisé poreux sous forme de granulés de taille minimum d'au moins 1 mm, associé à des fibres d'os cortical déminéralisé d'au moins 5 mm, et à un matériau coagulé, préférentiellement issu de moelle osseuse. Tous les exemples proposés comprennent des cellules de moelle osseuse.

L'invention permet de remédier aux inconvénients de l'art antérieur, et notamment, elle permet d'obtenir un biomatériau implantable à partir d'un support de synthèse, donc facile à produire avec des propriétés constantes et homogènes, et de sang coagulé, sans qu'il soit nécessaire de faire appel à des étapes de culture, ce matériau ayant une excellente biocompatibilité, permettant la reconstruction du tissu osseux de façon rapide. L'invention permet également l'obtention d'un os d'excellente qualité en termes de dureté et de vascularisation. En outre le procédé de fabrication de ce biomatériau est simple, facile à mettre en oeuvre, ne nécessite pas de multiples interventions sur l'individu à traiter, est peu coûteux comparativement aux procédés de l'art antérieur.

Le biomatériau de l'invention est sous forme d'une pâte qui comprend au moins un phosphate de calcium biphasé sous forme de granulés, mélangé de façon sensiblement homogène avec du sang coagulé.

Le phosphate de calcium biphasé, BCP, est utilisé dans de nombreuses applications médicales et dentaires. Le phosphate de calcium biphasé a été décrit pour la première fois comme matériau de réparation osseuse par Nery EB et al., J. Periodontol. 1992 Sept., 63(9) : 729-35. Le BCP est constitué d'un mélange d'hydroxyapatite (HA) Ca₁₀(PO₄)₆(OH)₂ et de phosphate tricalcique bêta (Ca₃(PO₄)₂) (β-TCP). Sa bioactivité et sa biorésorbabilité peuvent être contrôlées par la proportion d'hydroxyapatite et de β-TCP qui le constituent.

Les biomatériaux à base de BCP ont l'avantage, par rapport aux autres biomatériaux de synthèse, de favoriser l'ostéogenèse.

Le BCP a fait l'objet de nombreuses études : Fellah B.H. et al., J. Mater. Sci. : Mater. Med. (2007), 18, 287-294, ont montré que le choix d'une granulométrie inférieure à 20 µm favorisait la réponse inflammatoire des tissus, ce qui pourrait expliquer le fait que cette taille de particules soit particulièrement favorable à l'ostéogenèse, comme observé par Malard O. et al., J. Biomed. Mater. Res., 46(1), 1999, 103.

Mankani M.H. et al., Biotechnology and Bioengineering, 72(1), 2001, 96-107, ont au contraire montré que les particules de BCP calibrées d'une taille allant de 100 à 250 µm étaient celles qui entraînaient la plus importante reconstruction osseuse lorsqu'elles sont associées à des cellules de moelle osseuse cultivées, tandis qu'aucune formation osseuse n'était observée en dessous de 44 µm, et que de bons résultats sont obtenus avec des particules de taille allant jusqu'à 2 mm.

Trojani C. et al., Biomaterials, 27, 2006, 3256-3264, ont montré qu'une bonne ostéoinduction pouvait être obtenue par l'implantation d'un matériau composite BCP/hydrogel de Si- hydroxypropylméthyl cellulose auquel ont été additionnées des cellules de moelle osseuse cultivées, avec des particules de BCP calibrées de 40 à 80 µm.

Ces deux derniers procédés requièrent cependant une étape de prélèvement de cellules de moelle osseuse ainsi que leur mise en culture.

La présente invention se fonde sur les faits suivants :
- l'observation par les inventeurs que le BCP était doté de propriétés anticoagulantes,
- l'observation par les mêmes inventeurs qu'un BCP avec une granulométrie choisie associé à du sang coagulé, ou à un aspirat de moelle osseuse coagulé, permet d'obtenir une très bonne ostéogenèse et conduit à un tissu osseux de qualité très satisfaisante, à l'aide d'un procédé d'une très grande simplicité par rapport à ceux de l'art antérieur.

L'invention a donc pour premier objet l'association d'un BCP particulier défini ci-dessous avec du sang coagulé ou avec un aspirat de moelle osseuse coagulé. De façon avantageuse, cette association est sous forme d'une pâte homogène malléable.

Cette pâte peut être manipulée pour l'adapter à la taille et à la forme du défaut à combler, tout en veillant à ne pas lui appliquer de pressions excessives qui endommageraient ou détruiraient sa structure tridimensionnelle.

Le BCP utilisé dans la présente invention présente une granulométrie comprise entre 40 et 500 µm, préférentiellement entre 40 et 400 µm, encore plus préférentiellement entre 40 et 300 µm, et avantageusement entre 80 et 200 µm.

Le BCP utilisé dans l'invention consiste en un fritté haute température, broyé et calibré, par exemple par tamisage, en granulés de diamètre choisi. De façon avantageuse le BCP utilisé dans l'invention comporte de l'hydroxyapatite et du phosphate tri calcique β dans un rapport en poids/poids HA/ β-TCP compris entre 5/95 et 95/5, de préférence entre 30/70 et 80/20, avantageusement entre 40/60 et 60/40.

Avantageusement il s'agit d'un BCP poreux, avec des tailles de pores allant de 50 nm à 150 µm, préférentiellement de 1 µm à 50 µm.

Les granulés ou la poudre de phosphate tri calcique et d'hydroxyapatite peuvent être obtenus conformément aux méthodes décrites par Bouler et al., J Biomed Mater Res, 1996, 32, 603-609 ; Bouler et al., J Biomed Mater Res, 2000, 51, 680-684 ; Obadia et al., J Biomed Mater Res, 2006, 80(B), 32-42. Ils sont disponibles commercialement auprès de la société GRAFTYS SARL.

Si l'on applique les procédés de l'art antérieur au granulat de BCP, c'est-à-dire si l'on mélange le BCP avec un échantillon sanguin par exemple, on n'obtient pas de coagulat de sang car le BCP est doté de propriétés anticoagulantes. Selon le procédé de l'invention, le BCP est mélangé à un échantillon de sang préalablement prélevé sur anticoagulant ou prélevé sans anticoagulant et mis immédiatement au contact du BCP chez un donneur compatible avec le receveur du biomatériau, puis au moins un agent coagulant est additionné au mélange sous agitation. De préférence l'agent coagulant est un dérivé du calcium. Avantageusement l'agent coagulant à base de calcium est choisi parmi les sels de calcium biocompatibles comme CaCl₂, Ca(NO₃)₂, Ca(AcOEt)₂, CaSO₄.

Parmi les autres agents coagulants utilisables pour la mise en oeuvre de l'invention on peut citer la thrombine. Le mélange de BCP, de sang, ou d'aspirat de moelle, et d'agent coagulant est poursuivi pendant toute l'étape de coagulation et est d'une intensité adaptée pour permettre la formation d'un mélange homogène de granulés ou de particules de BCP et de sang coagulé, ou de moelle coagulée, et notamment le maintien en suspension des particules de BCP. Si cette agitation est trop ou pas assez forte, elle ne permet pas l'obtention d'un mélange homogène. L'homme du métier peut contrôler visuellement la formation d'un mélange homogène.

Outre le BCP, la composition du biomatériau peut comprendre des additifs éventuels tels que : polymères, particules de céramiques, molécules pharmaceutiques, les conditions pour l'emploi de ces matériaux étant : leur biocompatibilité, l'absence d'effet négatif sur la réaction de prise du biomatériau. De tels additifs bien connus de l'homme du métier sont destinés à modifier la rhéologie du biomatériau, son comportement *in vivo* (dureté, résorption, ostéogenèse) ou à agir sur l'apparition d'infections ou de phénomènes inflammatoires (antibiotiques, anti-infectieux, agents anti-inflammatoires).

On peut aussi prévoir d'introduire dans le biomatériau de l'invention des principes actifs, tels que des molécules thérapeutiques comme des molécules destinées à prévenir ou traiter une pathologie choisie par exemple parmi : un cancer, l'ostéoporose.

On peut également introduire dans le biomatériau de l'invention des facteurs de croissance, naturels ou de synthèse. On peut aussi prévoir la présence de biomarqueurs ou d'agents de contraste qui favorisent la visualisation par imagerie médicale de la résorption du biomatériau et son devenir dans l'organisme.

On peut prévoir d'introduire dans le biomatériau de l'invention du tissu adipeux, ou toute autre préparation de tissu ou de cellules, prélevé chez le patient auquel est destiné le biomatériau, ce tissu ou cette préparation ayant été préalablement mis en suspension dans du sang ou dans du plasma ou dans du sérum physiologique. Parmi les préparations tissulaires ou cellulaires on peut citer du tissu adipeux, des plaquettes, des cellules de moelle osseuse.

On peut aussi prévoir la présence de BCP de granulométrie différente, mais de préférence en petite quantité, avantageusement < 5% en poids/poids total de BCP, car il a été constaté que le contrôle de la granulométrie permettait une meilleure formation de tissu osseux (plus rapide, de meilleure qualité), et une bonne résorption.

Selon le procédé de l'invention, le BCP est placé dans une cavité d'un récipient fermé et stérile comme la cavité intérieure d'une seringue. Du sang ou de l'aspirat de moelle osseuse, préalablement prélevé sur un donneur compatible avec le receveur est introduit dans ce récipient.

Si le sang ou l'aspirat de moelle osseuse prélevé doit être stocké pendant une durée supérieure à quelques secondes (5 à 10 secondes), il est mélangé aussitôt après son prélèvement avec un anticoagulant afin d'éviter sa coagulation prématurée. Le sang du donneur peut par exemple être prélevé directement dans un tube contenant la quantité appropriée d'agent anticoagulant.

L'anticoagulant peut être un chélateur des ions calcium, comme par exemple du citrate de sodium, mais aussi de l'héparine par exemple.

Le mélange de BCP et de sang ou de moelle osseuse est effectué dans les proportions préférentielles suivantes :
de 10 à 90% en poids de BCP par rapport au volume de sang (ou de moelle osseuse), de préférence de 50 à 90%, et encore plus préférentiellement de 60 à 80%, en g/ml.

De façon avantageuse, le sang est prélevé chez le receveur lui-même de façon à garantir au mieux la biocompatibilité de l'implant. Selon une variante de l'invention le sang est remplacé par un produit dérivé du sang tel que du plasma. De préférence on utilise du sang total. Dans toute la demande y compris dans les revendications, lorsque l'on utilise le mot sang, on inclut dans sa définition les produits dérivés du sang tels que le plasma.

De façon préférentielle, dans la mise en oeuvre de l'invention, on utilise du sang ou du plasma, dont le prélèvement ne nécessite pas une intervention chirurgicale.

Suivant une variante de l'invention, le sang peut être prélevé à l'aide d'une seringue dans le corps de laquelle a été préalablement placé le BCP et éventuellement des additifs.

Après un premier mélange du BCP et du sang, l'agent coagulant est, lui aussi, additionné au mélange, par exemple par aspiration à l'aide de la seringue si l'on a utilisé un tel dispositif.

Le récipient clos contenant le BCP, le sang et l'agent coagulant est aussitôt agité, de façon à permettre la formation d'un matériau homogène. Par exemple, si l'on effectue le mélange dans un tube ou dans le corps d'une seringue, on place le récipient dans un agitateur rotatif dont la vitesse est réglée en fonction de la granulométrie du BCP, de façon à ce que les particules de BCP restent en suspension pendant que se produit la coagulation. Selon une variante de l'invention, l'agitation peut être produite par des billes aimantées associées à un agitateur magnétique.

Selon une variante préférée de l'invention, le récipient clos contenant le mélange de BCP, de sang et d'agent coagulant est laissé au repos pendant la phase de coagulation sanguine de façon à laisser sédimenter le BCP et à former un implant saturé en BCP.

A l'issue de cette étape, le mélange est sous forme d'une pâte malléable, homogène, comportant un réseau tridimensionnel de fibrine emprisonnant des particules sanguines, du plasma et les autres molécules qui ont été introduites dans la composition.

Suivant le type de dispositif qui a été employé pour la préparation du biomatériau de l'invention celui-ci peut ensuite être appliqué à l'aide des moyens les plus adaptés à l'emplacement où un défaut osseux doit être comblé :
A l'aide d'un outil tel qu'une spatule sans toutefois perturber l'organisation tridimensionnelle de l'implant, ou à l'aide de la seringue ou d'un autre dispositif cylindrique dont l'extrémité aura été préalablement sectionnée pour former une ouverture adaptée à la rhéologie du biomatériau de l'invention.

Ainsi, un autre objet de l'invention est un procédé pour la fabrication d'un biomatériau, ce procédé comportant au moins les étapes suivantes :
(i) mélange d'un BCP sous forme de granulés de taille comprise entre 40 et 500 µm avec du sang, ou avec un aspirat de moelle osseuse, en proportions allant de 10 à 90% en poids de BCP par volume de sang ou de moelle,
(ii) ajout au mélange de l'étape (i) d'au moins un agent coagulant en quantité suffisante pour provoquer la coagulation (du sang ou de la moelle),
(iii) mélange dans des conditions favorisant l'homogénéisation du BCP pendant que se produit la coagulation.

Comme cela a déjà été évoqué, dans le procédé de l'invention, les étapes (i) à (iii) peuvent être mises en oeuvre dans la cavité interne d'une seringue ou dans un tube fermé à ses extrémités. L'agent coagulant peut être choisi parmi les dérivés du calcium, tels ceux qui ont été énumérés ci-dessus, ou parmi les autres agents coagulants tels que la thrombine par exemple.

L'invention a encore pour objet un procédé de comblement d'un défaut osseux ce procédé comportant les étapes énumérées ci-dessus et comportant en outre une étape d'application du biomatériau obtenu à l'étape (iii) dans l'espace où un défaut osseux a été constaté. Ce procédé peut en outre comprendre des étapes d'incision de tissu et de suture.

Suivant la taille et la configuration du défaut osseux, le comblement par le biomatériau de l'invention peut être associé à une ostéosynthèse qui permet de conférer au tissu atteint la résistance mécanique nécessaire pendant que se produit la reconstruction osseuse sur le site d'implantation du biomatériau de l'invention.

Comme les inventeurs l'ont constaté, l'implantation du biomatériau de l'invention a permis d'induire la formation de tissu osseux dans des délais courts (quelques semaines), ce tissu osseux étant très richement vascularisé.

Au contraire, il a été constaté que l'implantation d'un biomatériau obtenu par le même procédé avec un BCP de granulométrie inférieure à 40 µm ne permettait pas d'obtenir de formation de tissu osseux de qualité satisfaisante et dans des délais staisfaisants. Et l'implantation d'un biomatériau obtenu par le même procédé avec un BCP de granulométrie supérieure à 500 µm conduit à un implant dont la résorbabilité est moins bonne.

Un autre objet de l'invention est constitué par un kit pour la mise en oeuvre du procédé de l'invention, ce kit comprenant l'association d'un BCP de granulométrie comprise entre 40 et 500 µm, de préférence entre 40 et 400 µm, avantageusement entre 40 et 300 µm et encore plus préférentiellement de 80 à 200 µm, avec au moins un agent coagulant. De préférence l'agent coagulant est dérivé du calcium. Avantageusement l'agent coagulant est CaCl₂.

La quantité d'agent coagulant est calculée pour compenser l'effet anticoagulant du BCP et éventuellement de l'agent anticoagulant qui est associé au sang prélevé.

La concentration en agent coagulant dans le mélange de sang et de BCP doit de préférence être comprise entre 1 et 50 mM, encore plus préférentiellement entre 3 et 35 mM, en particulier dans le cas où l'agent coagulant est à base de calcium. On doit préférentiellement ajouter l'agent coagulant en solution aqueuse de façon à ne pas dépasser 2 volumes de solution d'agent coagulant par poids de BCP en ml/g.

La concentration de la solution d'agent coagulant peut varier de façon à respecter ces deux contraintes, et en utilisant de préférence une solution d'agent coagulant de concentration inférieur à 120 mM, notamment lorsque l'agent coagulant est un sel de calcium.

Si le sang est prélevé sur anticoagulant il faut compenser l'effet de l'agent anticoagulant et l'effet du biomatériau.

Par exemple, pour du sang prélevé sur citrate de sodium dans des conditions classiques (de marque Vacuette®, disponibles auprès de la société Greiner Bio-One, ou de marque Vacutainer®, disponibles auprès de la société Becton Dickinson) et dans les proportions de 50 mg de BCP et 100 µl de sang, nous ajoutons 1/5^{ème} du volume final (soit 20 µl) d'une solution de calcium à 80 mM (concentration finale 13,3 mM). La concentration de la solution peut aller jusqu'à 120 mM. Au-delà, on peut être en excès de calcium, ce qui inhibe à nouveau la coagulation.

Si le sang n'est pas prélevé sur anticoagulant, le sang est prélevé directement sur le biomatériau et le calcium est ajouté secondairement. Dans ce cas, on peut ajouter 1/5^{ème} du volume de sang d'une solution aqueuse de sel de calcium de concentration allant de 12 mM à 60 mM environ.

Une telle association peut être sous la forme d'un kit stérile comportant :
(a) un dispositif comportant une cavité intérieure stérile dans lequel est placé le BCP,
(b) un réservoir stérile comportant l'agent coagulant.

Le réservoir (b) peut faire partie du dispositif (a) ou être une entité distincte telle qu'un tube ou un flacon dans lequel on peut prélever l'agent coagulant pour le transférer dans la cavité intérieure du dispositif (a), ou une seringue permettant l'injection de l'agent coagulant dans la cavité où est placé le BCP.

Avantageusement le dispositif (a) comporte des moyens permettant l'introduction de sang dans la cavité interne : par exemple des moyens permettant le prélèvement d'un échantillon de sang soit directement sur un individu, soit à partir d'un réservoir, ou des moyens permettant l'injection d'un échantillon de sang dans la cavité intérieure de façon à permettre le mélange avec le BCP. On peut prévoir que le sang prélevé soit directement introduit dans la cavité comprenant le BCP ou qu'il soit prélevé dans le réservoir où est placé l'anticoagulant puis que l'ensemble soit transféré dans la cavité où se trouve le BCP. Dans le cas où l'on utilise un aspirat de moelle osseuse, on prévoit un moyen d'aspiration de la moelle osseuse.

La cavité intérieure du dispositif (a) est d'une taille permettant d'y introduire la quantité de sang ou de moelle nécessaire pour produire le biomatériau de l'invention, ainsi que les autres constituants du mélange tels que : agent coagulant, principes actifs, préparations de tissu ou de cellules.

De façon avantageuse également le dispositif (a) comporte des moyens permettant l'application du biomatériau dans la zone où un défaut osseux a été constaté.

Un tel dispositif peut être constitué d'un dispositif cylindrique tel qu'un tube ou une seringue comme cela est illustré dans la partie expérimentale.

On peut également prévoir d'utiliser un dispositif tel que celui décrit dans WO 02/068010 comportant un tube à l'intérieur duquel est conservé le BCP, dans lequel on injecte le sang et l'agent coagulant et qui peut se voir adapter un piston pour restituer le biomatériau une fois que celui-ci est formé.

On peut encore prévoir d'utiliser un dispositif du type Vacutainer®, c'est-à-dire des tubes sous vide auxquels on adapte une seringue permettant de faire un prélèvement d'une quantité prédéterminée de sang, ces tubes étant pré-conditionnés avec du BCP dans leur cavité interne.

Un autre objet de l'invention est constitué par un biomatériau implantable comportant un BCP sous forme de granules dont la taille a été définie ci-dessus, dispersé de façon sensiblement homogène dans un réseau tridimensionnel de protéines sanguines ou dans un réseau de protéines de moelle osseuse.

Avantageusement ce biomatériau comporte un BCP tel que défini ci-dessus et un coagulat de protéines sanguines (ou de moelle) sous forme d'un mélange sensiblement homogène ayant l'aspect d'une pâte malléable.

Par pâte malléable on entend une matière qui ne s'écoule pas d'elle-même, comme le ferait un liquide, mais dont la résistance mécanique est suffisamment faible pour pouvoir être modelée sous l'effet d'une pression exercée manuellement par un individu, éventuellement à l'aide d'un instrument tel qu'une spatule ou le piston d'une seringue.

Un tel biomatériau peut être utilisé pour la fabrication d'un implant osseux, qu'il s'agisse de combler une fracture, une perte de substance d'origine traumatique ou tumorale, un défaut consécutif à une intervention chirurgicale, ou d'aider à la mise en place d'une prothèse.

Le biomatériau peut être introduit, comme illustré dans les exemples, par une intervention chirurgicale dans la zone où un défaut osseux doit être comblé. Après incision, le biomatériau est implanté et l'incision est refermée.

Le biomatériau de l'invention peut être associé à une ostéosynthèse de résistance mécanique plus élevée, de façon à permettre la stabilité du montage en attendant la colonisation de la zone déficiente par les tissus osseux.

On peut prévoir de l'associer à une prothèse. Un enrobage de la prothèse par le biomatériau de l'invention permet de favoriser l'implantation de tissu osseux vivant dans ou autour de la prothèse.

Le biomatériau de l'invention peut encore être utilisé *in vitro* ou *ex-vivo* comme support pour la production de tissu osseux :
En effet, la culture de cellules osseuses autour de ce biomatériau permet de produire un tissu osseux ultérieurement implantable.

Un autre objet de l'invention est l'utilisation *in vitro* ou *ex-vivo* d'un biomatériau tel que décrit ci-dessus pour produire un implant osseux.

On peut, selon l'invention cultiver des cellules osseuses sur le biomatériau de l'invention dans un moule ayant la forme de l'implant que l'on souhaite fabriquer. La culture de cellules dans ces conditions permet d'obtenir un implant biocompatible de forme et de dimensions adaptées.

### PARTIE EXPÉRIMENTALE

### Figures

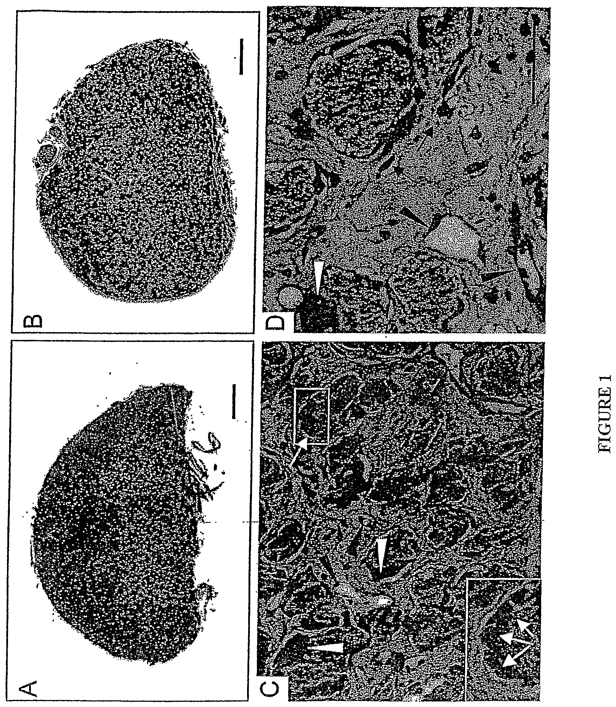

Figure 1 : Formation de tissu osseux par un implant de sang coagulé autour de particules de BCP.
Coupes transversales d'implants colorés au HES après 4 semaines d'implantation en site sous-cutané (A et C) et intramusculaire (B et D).

| | |
|---|---|
| Echelle : | AetB: 500 µm |
| | C et D : 50 µm |
| Flèches blanches : ostéoblastes | |
| Flèches noires : ostéocytes | |
| Têtes de flèches noires : vaisseaux sanguins | |
| Tête de flèche blanches : ostéoclastes | |

Figure 2 : Coupe d'implants sang/BCP après 4 semaines d'implantation.
(A) : hybridation par du sérum immun montrant la coloration burne d'ostéocalcine dans le cytoplasme des cellules (flèche blanche)
(B) : hybridation par du sérum non immun - Echelle : 10µm
(C) : Coloration Goldner - Echelle : 50 µm
Flèches noires : vaisseaux et ostéocytes.

Figure 3 : Microscopie électronique à balayage d'implants sang/BCP de 4 semaines
(A) matrice de collagène dans l'espace intergranulaire - Echelle : 10 µm
(B) grossissement de (A) - Echelle : 1 µm
(C) deux ostéoclastes attachés aux granules avec 2 à 3 noyaux visibles - Echelle : 10 µm
(D) capilaire fonctionnel - Echelle : 10 µm

Figure 4 : Microscopie électronique à balayage d'implants
(A) BCP/sang coagulé
(B) BCP/plasma coagulé
Echelle : 1 µm

Figure 5 : Formation de tissu osseux à partir d'implants BCP/plasma après 4 semaines d'implantation
Sites sous-cutanés (A, C)
Sites intramusculaires (B, D)

| | |
|---|---|
| Echelle : | 500 µm (A, B) |
| | 50 µm (C, D) |

Figure 6 : Comparaison des propriétés ostéogéniques du biomatériau préparé à partir de sang de souris C57BL/6 (A,B) et de sang humain (C,D) associés à des microparticules de BCP (40-80 µm), après implantation sous-cutanée chez des souris immunodéprimées de type nude. Observation à faible (A,C) et fort (B,D) grossissement.
Echelles : 100 µm.
Figure 7 : Influence de la taille des microparticules de BCP sur la formation osseuse après implantation sous-cutanée chez la souris. Des implants ont été préparés à partir de sang de souris C57BL/6 associé à du BCP sous forme (A) d'une grande quantité de poussière fine de taille inférieure à 40 µm mélangée à des particules de 80-200 µm ; (B) de particules de 40-80 µm ; (C,D) de particules de 80-200 µm ; (E,F) de particules de 200-500 µm. Les figures D et F correspondent à des vues à plus fort grossissement des implants C et E respectivement. Echelles 100 µm.
Figure 8 : implants préparés à partir de 100 µl de sang de souris C57BL/6 et de quantités croissantes de microparticules de BCP 40-80 µm : (A) 10 mg, (B) 30 mg, (C) 50 mg et (D) 70 mg. Echelle : 100 µm.
Figure 9 : radiographies chez le chien - implants préparés à partir de sang total coagulé autour de microparticules de BCP calibrées (80-200 µm) : (A) BCP/sang avec un ratio de 50%, les microparticules étant maintenues en suspension dans le sang pendant la phase de coagulation (méthode n°1) ; (B) BCP à concentration maximale dans le sang, les microparticules sédimentant pendant la coagulation (méthode n°2). Les flèches blanches indiquent la présence d'un liseré radiotransparent entre les extrémités diaphysaires et l'implant.
Figure 10 : Implantation chez le chien BEAGLE. Radiographies postopératoires. (A) à gauche l'implant est constitué de BCP seul. (B) à droite il est constitué du mélange BCP/sang avec un ratio maximum de BCP selon la méthode 2 de préparation des implants.

### 1. Principe :

Il s'agit d'une procédure extemporanée, réalisée au bloc opératoire. Elle consiste à mélanger dans le corps d'une seringue en polypropylène des particules de BCP et du sang total autologue (50% w/v) prélevé sur un anticoagulant chélateur des ions calcium. L'ajout de CaCl₂ permet de déclencher la coagulation. La seringue est alors placée pendant 10 minutes à température ambiante sur un mélangeur rotatif, ce qui permet de maintenir les particules de BCP en suspension dans le sang au fur et à mesure de la coagulation. On obtient ainsi une répartition homogène des particules au sein du sang coagulé. L'extrémité de la seringue est alors sectionnée et l'implant poussé hors de la seringue à l'aide du piston pour être placé dans le site d'implantation.

### Résultats obtenus chez l'animal (souris C57BL/6) :

L'implantation du biomatériau en site ectopique (sous cutané et intramusculaire) démontre ses propriétés ostéo-inductrices, avec des implants entièrement colonisés par un tissu osseux immature minéralisé très richement vascularisé après 4 semaines.

### 2. Matériels et Méthodes

### 2.1. Particules de phosphate de calcium biphasé :

Le biomatériau de phosphate de calcium biphasé (BCP) est composé de 60 % d'hydroxyapatite (HA; Ca₁₀(PO₄)₆(OH)₂) et de 40% de phosphate tricalcique (TCP; Ca₃(PO₄)₂). Les particules de BCP calibrées entre 80 et 200 microns ont été fournies par la société GRAFTYS SARL (Aix-en-Provence, France). Les particules ont été stérilisées par chauffage à 180°C pendant deux heures.

### 2.2. Préparation des implants et procédure chirurgicale :

### -Implantation sous-cutanée chez la souris

Les expériences ont été conduites en suivant la réglementation de la Direction des Services Vétérinaires et ont obtenu l'autorisation du Comité Régional d'Ethique pour l'Expérimentation Animale (CREEA). Le sang total est prélevé sur citrate de sodium (anticoagulation) par ponction intracardiaque à partir de souris C57BL/6 de dix semaines anesthésiées. Pour certaines expériences, le plasma a été préparé à partir du sang total par centrifugation à 1800g pendant 15 minutes.

Méthode 1 : Les implants sont préparés en mélangeant 100 µl de sang total (ou de plasma) avec 50 mg de particules de BCP dans une seringue de 1 ml. L'activation de la coagulation est ensuite obtenue par ajout de 20 µl d'une solution à 1% de CaCl₂. Durant la période de coagulation (5 à 10 minutes), la seringue est placée sur une roue de type New Brunswick "tissue culture roller, model TC-7 M1053-4005). Ceci permet un mouvement de rotation de la seringue sur elle-même et maintient les particules de BCP en suspension dans le caillot. Après section de l'extrémité de la seringue, les implants sont poussés hors de la seringue grâce au piston et implantés en sous-cutané (SC) ou en intramusculaire (IM) chez des souris C57BL/6.

Méthode 2 : Les implants sont préparés avec une concentration maximale en particules et donc un ratio BCP/sang maximum. Pour cela, le mélange BCP/ sang/calcium est maintenu en position fixe pendant la durée de la coagulation de façon à laisser les microparticules sédimenter naturellement dans le sang. On considère qu'on obtient ainsi une concentration maximale en microparticules dans le sang coagulé.

Les implants sous-cutanés ont été placés sous la peau en position dorsale et les implants intramusculaires ont été placés dans chaque cuisse entre les masses musculaires après dissection. Dans chacun des sites (SC et IM), on s'assure qu'aucun saignement n'a été induit au cours de l'implantation.

Dans chaque expérience d'implantation, les souris C57BL/6 sont anesthésiées par inhalation d'isoflurane à 4%. Deux implants SC et deux implants IM sont placés pour chaque souris. Dans certaines expériences, chaque souris a reçu un implant de sang/BCP et un implant de plasma/BCP à chaque site. Après 4 à 8 semaines, les animaux sont sacrifiés par inhalation de CO₂ et les implants prélevés pour analyse.

### - Implantation en site osseux chez le rat

Le protocole que nous avons appliqué chez le rat a été approuvé par le comité d'éthique régional pour l'expérimentation animale (NCA/2007/12-06). Ces expériences préliminaires, ont été réalisées à l'Animalerie Centrale de la Faculté de Médecine de Nice.

Nous avons utilisé un modèle de perte de substance osseuse, segmentaire, interruptrice, diaphysaire fémorale de taille critique (6 mm) associée à une ostéosynthèse par plaque - vis, modèle développé dans notre laboratoire. Nous opérons un seul fémur par rat, la perte de substance est comblée par notre biomatériau, sang total autologue/BCP (80-200 µm). Le suivi des rats est clinique (absence de douleur, déambulation, état général) et radiologique par des clichés à J0, J7, J15, J30, J45, J60, et J90. Au terme du troisième mois, les animaux sont sacrifiés, les fémurs sont prélevés et, après ablation du matériel d'ostéosynthèse, les os sont fixés dans du formol avant inclusion en résine de méthyl-métacrylate et étude histologique.

Lors des premières interventions, les implants ont été réalisés à la mesure du défect osseux, avec une proportion de particules de 50% poids/volume, soit 75 mg de BCP pour 150 µl de sang total prélevé sur l'animal en préopératoire. La coagulation est déclenchée grâce à l'ajout de 15 µl de CaCl₂ à 2% et l'homogénéisation est assurée par rotation continue jusqu'à coagulation afin de maintenir les particules de BCP en suspension dans le sang et assurer l'homogénéité du biomatériau.-

### - Implantation en site osseux chez le chien BEAGLE

Il s'agit d'un modèle de perte de substance osseuse cavitaire cylindrique calibrée de taille critique au niveau des condyles latéraux fémoraux, réalisée sur des chiens adultes de race Beagle. Pour chaque chien, les 2 condyles fémoraux ont été abordés. Le sang total (3,5 ml) est prélevé au début de l'intervention, par ponction au niveau de la veine jugulaire de l'animal et est utilisé pour préparer le biomatériau. La perte osseuse cylindrique de 8X10 mm est réalisée au niveau de chaque condyle fémoral externe et les séquestres osseux sont soigneusement éliminés par lavage au sérum physiologique et aspiration.

Chaque animal a reçu deux implants de composition différente mais préparés de façon à combler totalement le défaut créé, c'est-à-dire :
D'un côté le biomatériau à tester constitué par un mélange de BCP (80-200 µm) et de sang avec un ratio de 50% poids/volume soit 330 mg de BCP et 660 µl de sang total. Les particules de BCP sont maintenues en suspension pendant la coagulation par rotation de la seringue ayant servi à préparer le mélange.

De l'autre coté est implanté du BCP seul, hydraté dans du sérum physiologique soit 660 mg, ce qui occupe le même volume que l'implant controlatéral.

La durée de l'expérience est de 8 semaines.

Des radios ont été effectuées en post-opératoire immédiat et en fin d'expérience à l'ENVN.

### 2.3. Analyse histologique :

Les implants disséqués sont fixés pendant 24 heures dans une solution de formaline tamponnée à 10%. Chaque implant est ensuite sectionné en trois tranches qui sont décalcifiées ou non dans une solution de 10% (w/v) d'acide éthylènediaminetétracétique (EDTA) pendant 24 heures à température ambiante puis incluses en paraffine. Des sections de 4 µm sont réalisées, déparaffinées, hydratées et colorées à l'Hématoxyline, Erythrosine, Safran (HES). Les coupes sont ensuite observées en microscopie optique grâce à un microscope Zeiss Axioskop. Les photos sont réalisées grâce à une caméra couleur AxioCam HRc (Zeiss, Le Pecq, France). Afin de quantifier les surfaces occupées par l'os fibrillaire d'une part et par les particules de BCP d'autre part, chaque image d'implant a été subdivisée en trois zones de surface égale à 0,6 mm² selon l'axe médian de l'implant. Dans chacune de ces trois zones, l'aire occupée par le tissu osseux fibrillaire a été mesurée en utilisant le logiciel AxioVision Rel.4.6. Cette analyse a été effectuée pour trois implants SC et trois implants IM. Le nombre de vaisseaux et d'ostéoclastes a été évalué dans ces mêmes zones en comptant respectivement les capillaires et les cellules géantes multinucléées au microscope optique (100X) par deux observateurs différents. La densité en ostéoclastes et en vaisseaux est exprimée par mm² sous forme de moyenne + déviation standard. Le test statistique utilisé est le test T de Student. La significativité a été définie pour une valeur de p inférieure à 0,05.

### 2.4. Coloration Goldner :

Des coupes d'implants non décalcifiées de 7 µm d'épaisseur ont été colorées par la méthode Trichrome de Goldner qui permet d'évaluer la minéralisation du tissu osseux et de distinguer le tissu minéralisé (en bleu/vert) du tissu ostéoïde non minéralisé (en rouge). Brièvement, les sections déparaffinées sont réhydratées puis incubées en présence d'hématoxyline de Weigert pendant 20 min, rincées à l'eau courante et différentiées en présence d'alcool acide à 1%, lavées à l'eau courante pendant 5 min puis rincées à l'eau distillée. Les sections sont ensuite colorées par incubation dans une solution de Ponceau de xylidine/acide fuchsique/azophloxine/acide acétique pendant 5 min, rincées en acide acétique 1%, incubées en présence d'acide phosphomolybdique/orange G pendant 20 min, rincées en acide acétique 1%, colorées en présence de vert lumière/acide acétique pendant 5 minutes, lavées en 1% acide acétique pendant 5 minutes, séchées et montées en milieu de montage Entellan (Merck, Damstadt, Allemagne).

### 2.5. Immunohistochimie de l'ostéocalcine murine

Nous avons utilisé un anticorps polyclonal immunopurifié anti-ostéocalcine de souris, dirigé contre un peptide synthétique correspondant aux acides aminés 1-20 de l'extrémité N terminale de la protéine (Alexis Biochemicals, Lausanne, Suisse). Brièvement, des coupes en paraffine de 7µm ont été déparaffinées, réhydratées en éthanol, lavées en PBS et incubées 30 minutes en présence de H₂O₂ à 0,3% dans du PBS. Après deux lavages en PBS, les lames ont été incubées en présence de sérum de chèvre à 1,5% (tampon de blocage) pendant 30 min. Après lavage en PBS, l'incubation en présence d'un anticorps anti-immunoglobulines de lapin marqué à la biotine et la procédure de révélation à la peroxydase ont été réalisées en utilisant le kit de marquage ABC (sc-2118, Santa Cruz, CA, USA). Les sections ont été ensuite incubées en présence du substrat de la peroxydase pendant 10 min et les noyaux cellulaires colorés à l'hématoxyline pendant 3 min. Après déshydratation, le montage est réalisé en liquide de montage Entellan (Merck). Les contrôles sont réalisés en incubant les lames en présence de tampon de blocage.

### 2.6. Microscopie électronique à balayage

Les implants constitués de sang coagulé / BCP ou de plasma coagulé/BCP, avant et après quatre semaines d'implantation, ont été fixés pendant 12 heures à 4°C dans une solution de glutaraldéhyde tamponnée. Les échantillons ont été ensuite lavés et incubés en présence de glycérol à 30% pendant 1h puis congelés dans l'azote liquide et fracturés. Après déshydratation en présence de concentrations croissantes d'éthanol, ils ont été immergés dans l'hexaméthyldisilazane (Sigma-Aldrich, L'isle d'Abeau Chesnes, France) pendant 5 min puis séchés à température ambiante. Ils ont ensuite été fixés sur des supports en aluminium puis recouverts d'une couche d'or-palladium (Polaron E5100, UK). L'observation a ensuite été réalisée grâce à un microscope électronique à balayage de type JEOL 6700F (Japon).

### 3. Résultats

### 3.1. Analyse macroscopique et microscopique des implants sous-cutanés et intramusculaires de sang coagulé / BCP.

L'implantation de sang coagulé en absence de particules de BCP, n'a pas permis la formation d'un tissu osseux. Après quatre semaines, nous avons retrouvé uniquement une petite quantité de tissu fibreux au site d'implantation.

La dissection et l'examen macroscopique des implants de sang coagulé autour des particules de BCP après 4 et 8 semaines ont permis de constater leur consistance ferme ainsi que la présence de nombreux petits vaisseaux à leur surface. Aucune inflammation du tissu hôte n'a été observée.

L'analyse histologique des coupes en paraffine d'implants de sang / BCP après 4 semaines d'implantation a révélé une colonisation complète et reproductible de tout l'espace inter particulaire par du tissu osseux immature en contact étroit avec le BCP, à la fois pour les implants SC (Fig. 1A) et IM (Fig. 1B). L'observation à plus fort grossissement suggère que la matrice de collagène est plus mature en site IM (Fig. 1D) qu'en site SC (Fig. 1C). Pour évaluer la quantité d'os fibrillaire développée dans l'espace inter particulaire, la proportion entre les aires occupées par le tissu osseux et les aires occupées par le BCP a été calculée comme décrit dans les matériels et méthodes. Ceci a permis de montrer une différence significative entre les sites SC et IM avec 49,63 + 5,08 % de tissu osseux dans les implants IM et 42 + 8,33 % dans les implants SC (n = 9, p = 0,035). L'ensemble de ces résultats indique que l'espace inter particulaire est complètement colonisé dans les deux sites SC et IM, mais que la quantité de tissu développée est significativement plus grande dans les implants IM.

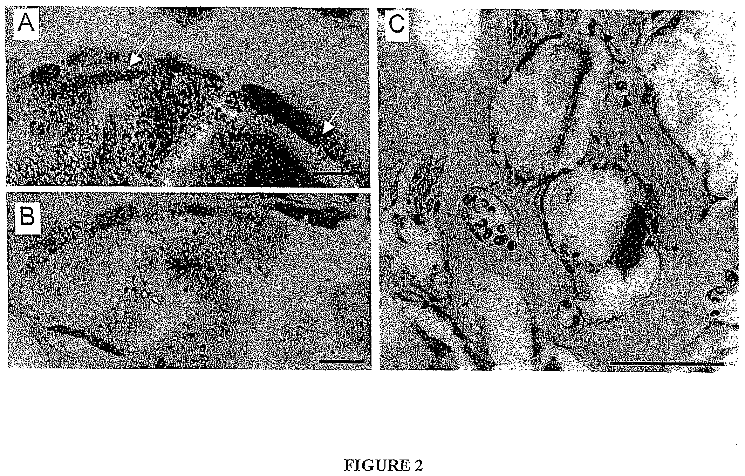

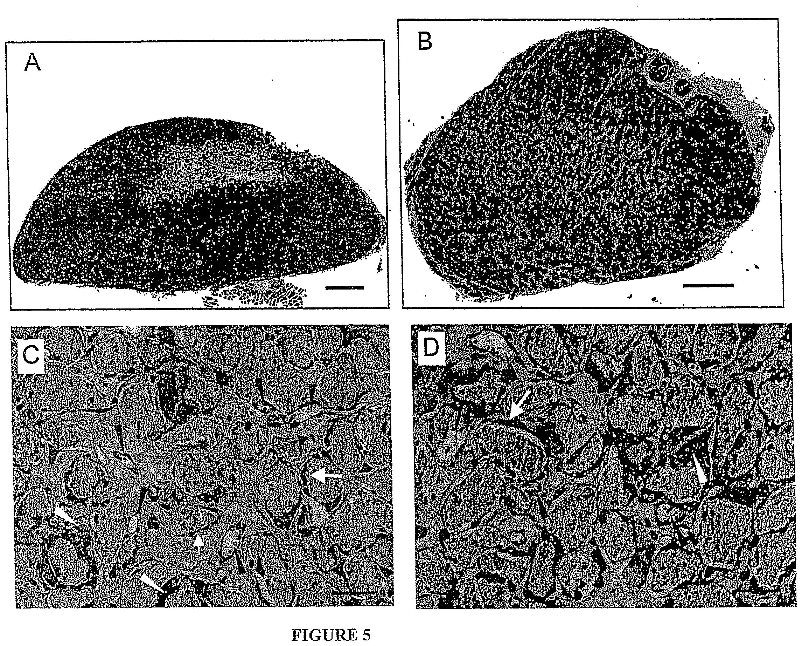

A chacun des sites, nous avons observé la présence de nombreux vaisseaux au sein de la matrice collagénique, répartis de façon homogène dans tous les implants (Fig 1C, D, têtes de flèches noires). Leur numération a révélé une différence significative entre les implants IM et SC avec une moyenne de 61,4 + 10,2 vaisseaux / mm² et 51,10 ± 10 / mm² respectivement (n = 9; p = 0,045). Nous avons également observé de nombreuses cellules géantes multinucléées attachées aux particules de BCP (têtes de flèches blanches). Ces cellules sont identiques à celles que nous avons identifiées comme étant des ostéoclastes dans un travail précédent (Trojani C. et al., Biomaterials, 27, 2006, 3256-3264). Leur numération a révélé une moyenne de 88,51+ 14,60 ostéoclastes / mm² dans les implants IM et de 93,13 + 14,40 / mm² dans les implants SC, différence non statistiquement significative. La forte capacité de résorption de ces cellules est fortement suggérée par la présence de micro particules et de cristaux de fragmentation intercytoplasmiques, par l'irrégularité du contour de certaines particules de BCP, leur texture de dégradation avec une densité plus basse et hétérogène. Enfin, nous avons observé la présence d'ostéoblastes cubiques alignés à la surface des particules de BCP (Fig. 1C et insert, Fig. 5C, flèches blanches) et de nombreuses cellules de type ostéocytaires incluses dans la matrice de collagène (Fig. 1D, 2B, 3A, B flèches noires). Le phénotype d'ostéoblaste mature de ces cellules a été démontré par la détection immunohistologique intracytoplasmique de l'ostéocalcine (Fig. 2A). De plus, tous les implants étaient positifs après coloration Goldner après 4 semaines d'implantation ce qui indique que ce tissu néoformé est minéralisé (Fig. 2C).

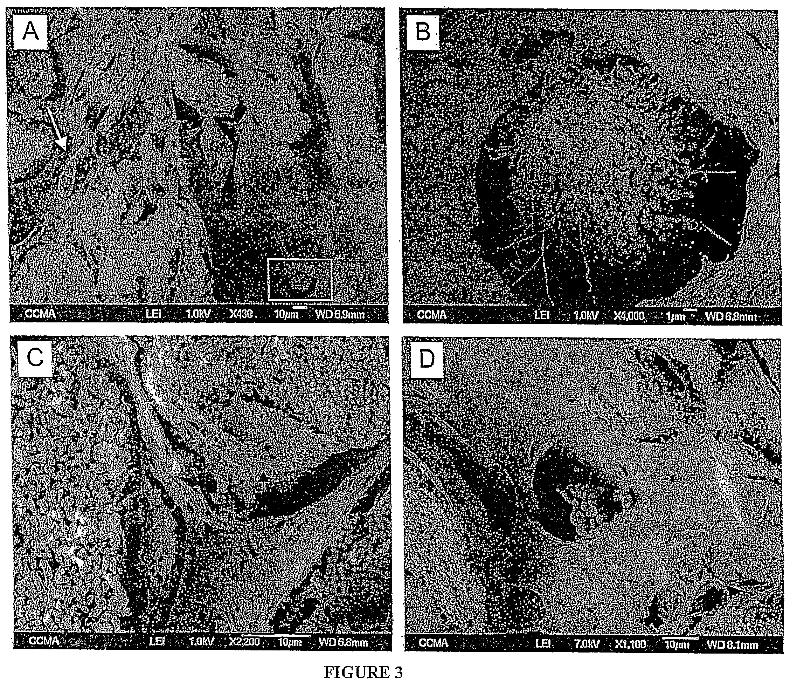

L'analyse des implants IM de 4 semaines en microscopie électronique à balayage a permis d'observer (Fig. 3) la microporosité des particules de BCP, la matrice de collagène remplissant les espaces inter particulaires, la présence de capillaires fonctionnels contenant des érythrocytes (Fig. 3A, D, flèche blanche) et d'ostéoclastes attachés aux granules de BCP (Fig. 3C, flèches noires). De plus, elle a confirmé la présence de cellules de type ostéocytaire, étoilées, possédant de nombreux prolongements irradiant dans toutes les directions, incluses dans la matrice de collagène et entourées par un espace péricellulaire de type ostéoplaste (Fig. 3A insert, 3B).

Les résultats obtenus après 8 semaines d'implantation n'ont pas révélé de différence significative avec les implants de 4 semaines. Tous les implants SC et IM sont entièrement colonisés par de l'os immature présentant les mêmes caractéristiques histologiques.

### 3.2. Analyse macroscopique et microscopique des implants SC et IM de plasma coagulé / BCP :

Afin d'analyser le rôle respectif du plasma et des cellules sanguines dans la néoformation osseuse nous avons implanté en parallèle pour chaque souris et à chaque site (SC et IM) un implant de sang coagulé / BCP et un implant de plasma coagulé / BCP.

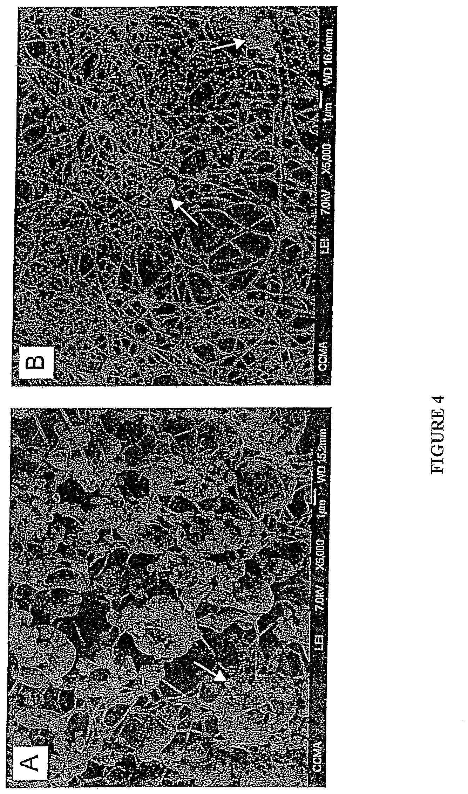

La structure du réseau de fibrine des deux types d'implant, sang coagulé / BCP et plasma coagulé / BCP, a été analysée en microscopie électronique à balayage. Ceci a montré que la maille de fibrine obtenue avec le sang coagulé était plus large que celle observée avec le plasma coagulé (Fig. 4A, B). Comme attendu, les globules rouges et les plaquettes sont les cellules prédominantes observées dans les implants de sang coagulé / BCP. La conservation de leur forme et de leur structure démontre une bonne viabilité (Fig 4A), ce qui indique que le mélange de sang et de particules de BCP n'a pas d'effet délétère sur les cellules sanguines.

La dissection et l'examen macroscopique des implants de plasma coagulé / BCP après 4 et 8 semaines a révélé des caractéristiques similaires à celles des implants de sang coagulé /BCP *i.e.* une consistance ferme et de nombreux vaisseaux de surface visibles (résultats non montrés). L'analyse histologique après 4 semaines a montré que les implants SC étaient tous colonisés à 80% environ (Fig 5A) par de l'os néoformé. L'analyse des implants IM a révélé une colonisation totale dans 75 % des cas (Fig. 5B) et dans 25% des cas, la présence d'une petite zone centrale de tissu plus fibreux et lâche (résultat non montré). Dans chacun des sites, le tissu osseux fibrillaire néoformé présentait les mêmes caractéristiques que celles du tissu obtenu dans les implants de sang coagulé / BCP (Fig 5C, 5D). En conclusion, ces résultats démontrent que l'utilisation du sang total permet d'obtenir une colonisation complète des implants dans les deux sites. L'association de plasma coagulé et de particules de BCP engendre une colonisation non complète.

### 3.3 Analyse macroscopique et microscopique d'implants composés de sang humain coagulé et de particules de BCP de 40-80 µm, après implantation sous-cutanée chez des souris immunodéprimées.

Nous avons analysé la formation osseuse induite par le biomatériau préparé à partir de sang humain coagulé autour de particules de BCP de 40-80 µm, après implantation à des souris immunodéprimées de type nude. En parallèle, sur les mêmes animaux, nous avons implanté le biomatériau préparé à partir de sang de souris C57BL/6 afin de comparer, sur le même animal receveur, la formation osseuse induite par du sang de souris et celle engendrée par du sang humain. Après 6 semaines d'implantation, les implants ont été prélevés, fixés et l'analyse histologique réalisée comme décrit précédemment.

Avant même l'étude histologique, nous avons constaté la dureté tout à fait inhabituelle des implants préparés à partir de sang humain alors que les implants de sang de souris étaient d'une consistance plus élastique.

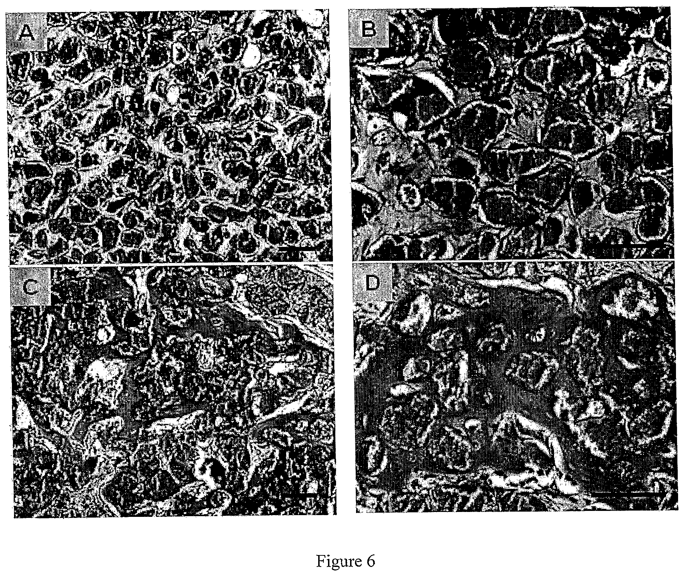

L'analyse histologique des implants murins a révélé un tissu osseux immature de qualité équivalente à celle retrouvée lors des expériences précédentes réalisées en système syngénique (implantation de sang de souris C57BL/6 sur des souris C57BL/6). Nous avons observé un tissu fibrillaire collagénique très vascularisé dans lequel on peut mettre en évidence des cellules osseuses, ostéoblastes, ostéocytes et ostéoclastes (Fig. 6A, 6B).

L'analyse histologique des implants humains a donné des résultats très différents avec colonisation par un tissu osseux mature. Les cellules osseuses sont moins nombreuses, au profit des ostéocytes. Le tissu de soutien présente une organisation en fibres de collagène mieux structurées, alignées et plus denses, avec en son sein des plages d'hématopoïèse caractérisées par la présence de cellules hématopoïétiques immatures comme les érythroblastes ainsi que des adipocytes (Fig. 6C, 6D). La dureté de ces implants à la coupe suggérait que ce tissu était fortement minéralisé. Nous avons donc obtenu un tissu osseux lamellaire mature.

La différence de maturité des tissus osseux obtenus après implantation des sangs humain et murin pourrait résulter de propriétés différentes du sang de ces deux espèces, propriétés liées à la composition cellulaire et/ou à la composition protéique, aux facteurs de croissance, aux facteurs solubles et au réseau de fibrine. Des expériences seront menées pour tenter de comprendre les mécanismes impliqués.

La comparaison de nos résultats avec ceux de la littérature a montré que le tissu osseux mature que nous avons obtenu à partir du sang humain était très similaire à celui que plusieurs groupes ont décrit après implantation de cellules stromales mésenchymateuses (MSC) humaines sélectionnées, amplifiées, différenciées *ex vivo* en ostéoblastes puis associées à de la poudre de BCP et implantées en sous-cutané à des souris immunodéprimées.

L'ensemble de ces résultats est donc très prometteur pour l'application clinique.

### 3.4 Influence de la granulométrie du BCP sur la formation osseuse

Nous avons testé 4 formes de BCP, trois formes microparticulaires calibrées respectivement entre 40 et 80 µm, 80 et 200 µm et 200 et 500 µm, ainsi qu'un mélange de particules de 80-200 µm et de fine poussière de taille très inférieure à 40 µm, la proportion de fine poussière étant de 40% en poids par rapport au poids total du mélange. Des implants ont été préparés dans les conditions déjà décrites, à partir de sang de souris C57BL/6 et de chacune de ces formes de BCP. La formation osseuse a été analysée après 8 semaines d'implantation sous-cutanée chez des souris syngéniques C57BL/6.

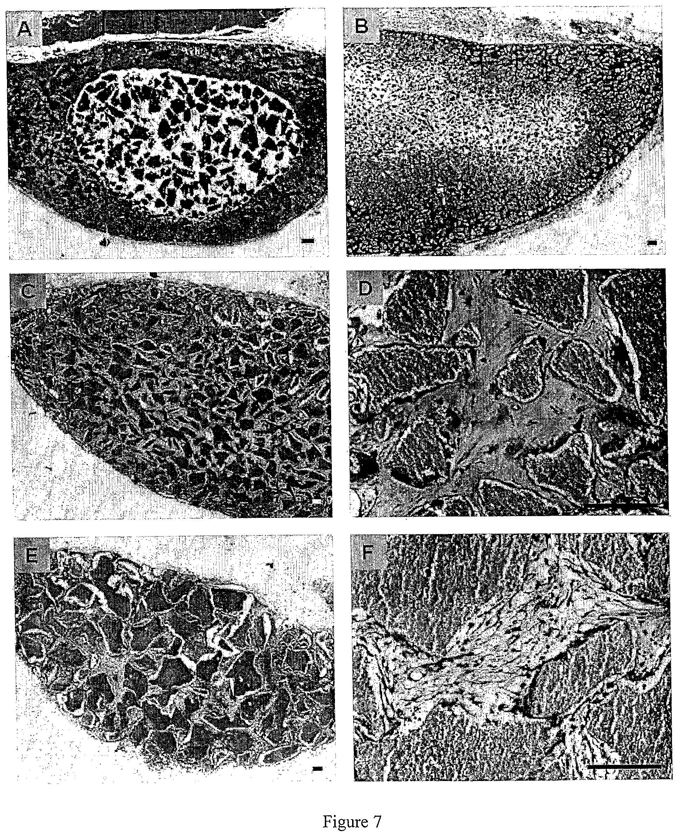

Les résultats de la figure 7 illustrent que les implants constitués de microparticules de 80-200 µm engendrent de façon très reproductible la meilleure colonisation par un tissu osseux immature ayant les caractéristiques déjà décrites (Fig. 7C, 7D). La présence de poussière de BCP (taille inférieure à 40 µm) mélangée aux microparticules de BCP de 80-200 µm est extrêmement délétère pour la formation osseuse comme on le voit sur la figure 7A. En effet on observe une colonisation des implants restant toujours strictement limitée à une couronne périphérique. Les implants constitués de grains 40-80 µm engendrent une bonne colonisation mais de façon moins reproductible que les implants 80-200 µm. En effet, de façon inexpliquée, on observe parfois un défaut de colonisation centrale comme on le voit sur la figure 7B. Enfin, les implants constitués de grains 200-500µm sont toujours colonisés mais par un tissu plus fibreux et lâche d'une qualité moins satisfaisante (Fig. 7E, 7F).

Ces résultats démontrent que, dans notre biomatériau, la granulosité 80-200 µm est la plus favorable à la formation osseuse en site ectopique.

### 3.5. Détermination du ratio BCP/sang optimal pour la reconstruction osseuse

Il est possible d'incorporer à notre biomatériau des quantités variables de microparticules de BCP pour un même volume de sang. La détermination du ratio idéal était une étape importante dans sa mise au point. Plusieurs expériences d'implantation en site sous-cutané chez la souris, ainsi que des expériences préliminaires réalisées en site osseux chez le rat WISTAR et chez le chien BEAGLE, nous ont permis de préciser les meilleurs ratio BCP/sang.

### 3.5.1. Implantation en site ectopique sous-cutané chez la souris

Nous avons préparé des implants constitués d'une quantité fixe de sang (100 µl), d'une quantité fixe de CaCl₂ (10 µl) et d'une quantité croissante de microparticules de BCP de 40-80 µm : 10 mg, 30 mg, 50 mg et 70 mg, soit des ratios BCP/sang de 10, 30, 50 et 70 % poids/volume. Les particules de BCP ont été maintenues en suspension dans le sang pendant la coagulation par rotation sur une roue (méthode 1). Ces implants possédaient des tailles équivalentes au moment de l'implantation, avec des volumes respectifs de : 112, 116, 120 et 124 µl.

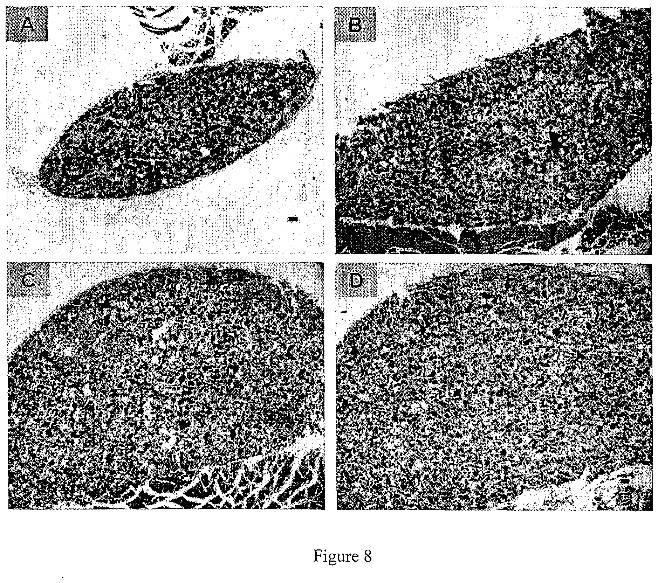

Comme on peut le voir sur la figure 8, après 4 semaines d'implantation, nous avons constaté que la taille finale des implants était proportionnelle au poids initial de BCP incorporé, et que tous les implants présentaient une densité particulaire équivalente. Par ailleurs, la colonisation par du tissu osseux immature était la même quel que soit le ratio BCP/sang.

Ces résultats indiquent que la dispersion homogène des grains dans l'implant initial, obtenue par rotation des seringues pendant la coagulation, ne se maintient pas au cours du temps. Au contraire il survient un phénomène de tassement des particules, probablement lié à la dégradation naturelle du gel de fibrine *in vivo.* Ce tassement aboutissant à la même concentration de grains pour un volume donné, quel que soit le ratio initial, il apparaît logique que le phénomène d'ostéoinduction soit le même.

Les expériences réalisées, ensuite, en site osseux ont confirmé ces résultats.

### 3.5.2. Implantation en site osseux chez le rat

Nous opérons un seul fémur par rat, la perte de substance est comblée par notre biomatériau, sang total autologue/BCP (80-200 µm). Le suivi des rats est clinique (absence de douleur, déambulation, état général) et radiologique par des clichés à J0, J7, J15, J30, J45, J60, et J90. Au terme du troisième mois, les animaux sont sacrifiés, les fémurs sont prélevés et, après ablation du matériel d'ostéosynthèse, les os sont fixés dans du formol avant inclusion en résine de méthyl-métacrylate et étude histologique.

Lors des premières interventions, les implants ont été réalisés à la mesure du défect osseux, avec une proportion de particules de 50% poids/volume, soit 75 mg de BCP pour 150 µl de sang total prélevé sur l'animal en préopératoire. La coagulation est déclenchée grâce à l'ajout de 15 µl de CaCl₂ à 2% et l'homogénéisation est assurée par rotation continue jusqu'à coagulation afin de maintenir les particules de BCP en suspension dans le sang et assurer l'homogénéité du biomatériau.

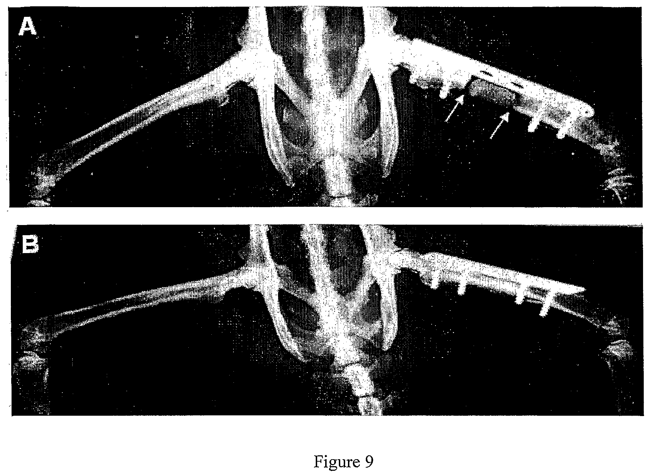

Nous avons observé de façon très reproductible et dès les premiers clichés radiologiques à J7, l'apparition d'un liseré radio transparent entre les berges osseuses et l'implant (fig. 9A), traduisant une absence de cohésion entre le biomatériau et les sections diaphysaires. Lorsque le BCP est à concentration maximale dans le sang, les microparticules sédimentant pendant la coagulation (méthode n°2), on observe une cohésion satisfaisante entre le biomatériau et les sections diaphysaires (figure 9B).

### 3.5.3. Comparaison des résultats obtenus avec les deux méthodes de préparation des implants.

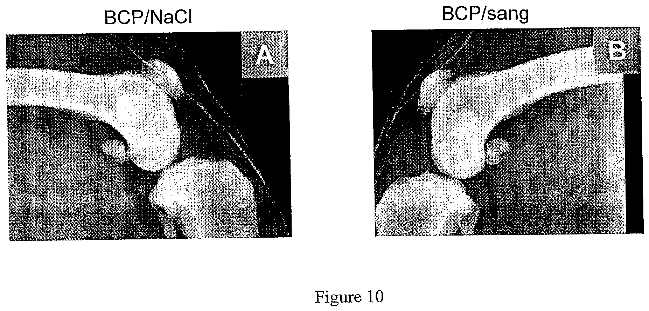

Chez le rat (Fig. 9B) et chez le chien (Fig. 10A et 10B) en site osseux, les analyses radiologiques ont montré, avec ce nouveau protocole, l'absence de rétrécissement des implants sur les radios de face ainsi que l'absence de liseré sur les radios de profil. Chez la souris en site ectopique, nous n'avons pas observé de différences de résultats sur la formation osseuse.

### 3.5.4. Discussion :

Dans le cas d'une implantation osseuse d'un biomatériau sédimenté (méthode 2), il n'y a pas de diminution des dimensions de l'implant, comme nous le constatons sur les radiographies effectuées sur les rats et les chiens, et s'il a été correctement mis en place, au contact étroit de l'os, il bénéficie des phénomènes d'ostéoconduction et d'ostéoinduction de façon optimale.

## Revendications

1. Procédé pour la fabrication d'un biomatériau ce procédé comportant au moins les étapes suivantes :
(i) mélange d'un phosphate de calcium biphasé, ou BCP, sous forme de granulés de taille comprise entre 40 et 500 µm avec du sang, ou avec un aspirat de moelle osseuse, en proportions allant de 10 à 90% en poids de BCP par volume de sang ou de moelle, en g/ml,
(ii) ajout au mélange de l'étape (i) d'au moins un agent coagulant en quantité suffisante pour provoquer la coagulation du sang ou de la moelle,
(iii) mélange dans des conditions favorisant l'homogénéisation du BCP pendant que se produit la coagulation.

2. Procédé selon la revendication 1, dans lequel l'agent coagulant à base de calcium est choisi parmi : les sels de calcium biocompatibles et de préférence CaCl₂.

3. Procédé selon l'une quelconque des revendications précédentes, dans lequel le sang a été préalablement prélevé chez un donneur compatible avec le receveur auquel est destiné le biomatériau.

4. Procédé selon l'une quelconque des revendications précédentes, dans lequel le sang a été préalablement prélevé chez le receveur auquel est destiné le biomatériau.

5. Procédé selon l'une quelconque des revendications précédentes, dans lequel le mélange contenant le BCP, le sang et l'agent coagulant est laissé au repos pendant la phase de coagulation sanguine de façon à laisser sédimenter le BCP et à former un implant saturé en BCP.

6. Procédé selon l'une quelconque des revendications précédentes, dans lequel les étapes (i) à (iii) sont mises en oeuvre dans la cavité interne d'une seringue ou dans un tube fermé à ses extrémités.

7. Procédé selon l'une quelconque des revendications précédentes, qui comporte le mélange de 50 à 90% en poids de BCP par rapport au volume de sang en g/ml, de préférence de 60 à 80%.

8. Biomatériau susceptible d'être obtenu par le procédé selon l'une quelconque des revendications 1 à 7 comprenant un BCP sous forme de granulés de taille comprise entre 40 et 500 µm, avantageusement de 80 à 200 µm, et du sang coagulé ou de la moelle coagulée.

9. Biomatériau selon la revendication 8 sous forme d'une pâte homogène malléable.

10. Biomatériau selon l'une quelconque des revendications 8 et 9 dans lequel le BCP comporte de l'hydroxyapatite (HA) et du phosphate tri calcique β (β-TCP) dans un rapport en poids/poids HA/ β-TCP compris entre 5/95 et 95/5.

11. Biomatériau selon l'une quelconque des revendications 8 à 10 et comprenant en outre au moins un additif choisi parmi : des polymères, des particules de céramiques, des molécules pharmaceutiques, des facteurs de croissance, naturels ou de synthèse, des biomarqueurs, des agents de contraste, des préparations de tissu ou de cellules.

12. Biomatériau selon l'une quelconque des revendications 8 à 11 pour son utilisation comme implant dans un procédé de comblement d'un défaut osseux.

13. Association d'un biomatériau selon l'une quelconque des revendications 8 à 12 avec une ostéosynthèse.

14. Kit pour la mise en oeuvre du procédé selon l'une quelconque des revendications 1 à 7, ce kit comprenant l'association d'un BCP de granulométrie comprise entre 40 et 500 µm avec un agent coagulant dérivé du calcium.

15. Kit selon la revendication 14 dans lequel l'agent coagulant est CaCl₂.

16. Kit selon l'une quelconque des revendications 14 et 15 qui comporte :
(a) un dispositif comportant une cavité intérieure stérile dans lequel est placé le BCP,
(b) un réservoir stérile comportant l'agent coagulant.

17. Kit selon l'une quelconque des revendications 14 à 16 dans lequel le dispositif (a) comporte des moyens permettant l'introduction de sang dans la cavité intérieure.

18. Kit selon l'une quelconque des revendications 14 à 17 dans lequel le dispositif (a) comporte des moyens permettant l'application du biomatériau dans la zone où un défaut osseux a été constaté.

19. Kit selon l'une quelconque des revendications 14 à 18 dans lequel le dispositif (a) est constitué d'une seringue.

20. Utilisation d'un biomatériau selon l'une quelconque des revendications 8 à 12 *in vitro* ou *ex-vivo* comme support pour la production de tissu osseux, ou pour produire un implant osseux.

## Patentansprüche

1. Verfahren zur Herstellung eines Biomaterials, wobei das Verfahren mindestens die folgenden Schritte beinhaltet:
(i) Mischen von zweiphasigem Calciumphosphat oder BCP in Form von Granulaten, die eine Größe zwischen 40 und 500 µm besitzen, mit Blut oder mit einem Knochenmarkaspirat in Anteilen von 10 bis 90 Gew.-% BCP pro Volumen Blut oder Mark in g/ml,
(ii) Zugabe zu dem Gemisch von Schritt (i) mindestens eines Gerinnungsmittels in einer Menge, die ausreicht, um die Gerinnung des Bluts oder des Marks hervorzurufen,
(iii) Mischen unter Bedingungen, die die Homogenisierung des BCP begünstigen, während die Gerinnung stattfindet.

2. Verfahren nach Anspruch 1, wobei das Gerinnungsmittel auf der Basis von Calcium aus Folgenden ausgewählt ist: biokompatiblen Calciumsalzen und vorzugsweise CaCl₂.

3. Verfahren nach einem der vorhergehenden Ansprüche, wobei das Blut zuvor von einem Spender entnommen wird, der mit dem Empfänger, für den das Biomaterial bestimmt ist, kompatibel ist.

4. Verfahren nach einem der vorhergehenden Ansprüche, wobei das Blut zuvor von dem Empfänger entnommen wurde, für den das Biomaterial bestimmt ist.

5. Verfahren nach einem der vorhergehenden Ansprüche, wobei das Gemisch, das BCP, Blut und Gerinnungsmittel enthält, während der Blutgerinnungsphase ruhen gelassen wird, damit sich das BCP absetzen und ein an BCP gesättigtes Implantat bilden kann.

6. Verfahren nach einem der vorhergehenden Ansprüche, wobei die Schritte (i) bis (iii) in dem inneren Hohlraum einer Spritze oder in einem an seinen Enden verschlossenen Röhrchen durchgeführt werden.

7. Verfahren nach einem der vorhergehenden Ansprüche, das das Mischen von 50 bis 90 Gew.-% BCP, bezogen auf das Volumen an Blut in g/ml, vorzugsweise von 60 bis 80 Gew.-%, beinhaltet.

8. Biomaterial, das durch das Verfahren nach einem der Ansprüche 1 bis 7 erhalten werden kann, umfassend ein BCP in Form von Granulat einer Größe zwischen 40 und 500 µm, vorteilhafterweise 80 bis 200 µm, und geronnenes Blut oder geronnenes Mark.

9. Biomaterial nach Anspruch 8 in Form einer homogenen formbaren Paste.

10. Biomaterial nach einem der Ansprüche 8 und 9, wobei das BCP Hydroxyapatit (HA) und β-Tricalciumphosphat (β-TCP) in einem Verhältnis Gewicht/Gewicht von HA/β-TCP zwischen 5/95 und 95/5 umfasst.

11. Biomaterial nach einem der Ansprüche 8 bis 10 und außerdem umfassend mindestens einen Zusatzstoff, ausgewählt aus: Polymeren, Keramikpartikeln, pharmazeutischen Molekülen, natürlichen oder synthetischen Wachstumsfaktoren, Biomarkern, Kontrastmitteln, Gewebepräparationen oder Zellen.

12. Biomaterial nach einem der Ansprüche 8 bis 11 für die Verwendung als Implantat bei einem Verfahren zum Auffüllen eines Knochendefekts.

13. Kombination eines Biomaterials nach einem der Ansprüche 8 bis 12 mit einer Osteosynthese.

14. Kit zur Durchführung des Verfahrens nach einem der Ansprüche 1 bis 7, wobei das Kit die Kombination eines BCP, das eine Partikelgröße zwischen 40 und 500 µm besitzt, mit einem von Calcium stammenden Gerinnungsmittel umfasst.

15. Kit nach Anspruch 14, wobei das Gerinnungsmittel CaCl₂ ist.

16. Kit nach einem der Ansprüche 14 und 15, das Folgendes enthält:
(a) eine Vorrichtung mit einem sterilen inneren Hohlraum umfasst, in den das BCP platziert ist,
(b) einen sterilen Behälter, der das Gerinnungsmittel enthält.

17. Kit nach einem der Ansprüche 14 bis 16, wobei die Vorrichtung (a) Mittel enthält, die die Einführung von Blut in den inneren Hohlraum ermöglichen.

18. Kit nach einem der Ansprüche 14 bis 17, wobei die Vorrichtung (a) Mittel enthält, die die Applikation des Biomaterials in dem Bereich, in dem ein Knochendefekt festgestellt wurde, gestattet.

19. Kit nach einem der Ansprüche 14 bis 18, wobei die Vorrichtung (a) aus einer Spritze besteht.

20. Verwendung eines Biomaterials nach einem der Ansprüche 8 bis 12 *in vitro* oder *ex vivo* als Träger für die Produktion von Knochengewebe oder zum Herstellen eines Knochenimplantats.

## Claims

1. Method for the manufacture of a biomaterial, this method comprising at least the following steps:
(i) mixing a biphasic calcium phosphate, or BCP, in the form of granules whose size is between 40 and 500 µm with blood, or with a bone marrow aspirate, in a proportion ranging from 10 to 90% by weight of BCP per volume of blood or marrow, in g/ml,
(ii) adding to the mixture of step (i) at least one coagulating agent in a sufficient quantity to cause the coagulation of the blood or of the marrow,
(iii) mixing under conditions promoting the homogenization of the BCP while the coagulation occurs.

2. Method according to Claim 1, in which the coagulating agent based on calcium is chosen from: biocompatible calcium salts and preferably CaCl₂.

3. Method according to either of the preceding claims, in which the blood was collected beforehand from a donor compatible with the recipient for whom the biomaterial is intended.

4. Method according to any one of the preceding claims, in which the blood was collected beforehand from the recipient for whom the biomaterial is intended.

5. Method according to any one of the preceding claims, in which the mixture containing the BCP, the blood and the coagulating agent is allowed to stand during the blood coagulation phase so as to allow the BCP to sediment and to form an implant saturated with BCP.

6. Method according to any one of the preceding claims, in which steps (i) to (iii) are carried out in the inner cavity of a syringe or in a tube closed at its ends.

7. Method according to any one of the preceding claims, which comprises mixing from 50 to 90% by weight of BCP relative to the volume of blood in g/ml, preferably from 60 to 80%.

8. Biomaterial which can be obtained by the method according to any one of Claims 1 to 7, comprising a BCP in the form of granules having a size of between 40 and 500 µm, advantageously from 80 to 200 µm, and coagulated blood or coagulated marrow.

9. Biomaterial according to Claim 8, in the form of a malleable homogeneous paste.

10. Biomaterial according to either of Claims 8 and 9, in which the BCP contains hydroxyapatite (HA) and β-tricalcium phosphate (β-TCP) in an HA/β-TCP weight/weight ratio of between 5/95 and 95/5.

11. Biomaterial according to any one of Claims 8 to 10 and additionally comprising at least one additive chosen from: polymers, ceramic particles, pharmaceutical molecules, growth factors, natural or synthetic, biomarkers, contrast agents, tissue or cell preparations.

12. Biomaterial according to any one of Claims 8 to 11, for its use as implant in a method for filling in a bone defect.

13. Combination of a biomaterial according to any one of Claims 8 to 12 with an osteosynthesis.

14. Kit for carrying out the method according to any one of Claims 1 to 7, this kit comprising the combination of a BCP having a particle size of between 40 and 500 µm with a coagulating agent derived from calcium.

15. Kit according to Claim 14, in which the coagulating agent is CaCl₂.

16. Kit according to either of Claims 14 and 15, which comprises:
(a) a device comprising a sterile inner cavity in which the BCP is placed,
(b) a sterile reservoir comprising the coagulating agent.

17. Kit according to any one of Claims 14 to 16, in which the device (a) comprises means allowing the introduction of blood into the inner cavity.

18. Kit according to any one of Claims 14 to 17, in which the device (a) comprises means allowing the application of the biomaterial in the zone where a bone defect has been observed.

19. Kit according to any one of Claims 14 to 18, in which the device (a) consists of a syringe.

20. Use of a biomaterial according to any one of Claims 8 to 12 *in vitro* or *ex vivo* as support for the production of bone tissue, or for producing a bone implant.
